# EUROPEAN PATENT APPLICATION

(11) **EP 1 074 185 A1**
(43) Date of publication of application: **07.02.2001**
(21) Application number: 00306317.9
(22) Date of filing: 25.07.2000
(51) Int. Cl.: A23L 1/30, A61K 9/20, A61K 31/575, A61P 3/06

(54) **Incorporation of cholesterol lowering agents into confectionery dosage forms**

(30) Priority: 26.07.1999 US 360691
(71) Applicant: McNeil-PPC, Inc., Skillman, NJ 08558-9418 (US)
(72) Inventor: Buehler, Gail K., Lower Gwynedd, PA 19002 (US); Higgins, John D., Ft. Washington, PA 19034 (US)
(74) Representative: Mercer, Christopher Paul

(57) **Abstract**

The present invention provides a cholesterol reducing agent in a confectionery dosage form. In a preferred embodiment the confectionery product is prepared in the absence of vegetable oils and fat. A method for preparing the confectionery forms is also disclosed.

## Description

### Field of the Invention

This invention relates to the incorporation of cholesterol-lowering agents into confectionery dosage forms.

### Background of the Invention

It has been shown that the addition of plant sterols (i.e., β-sitosterol) to diets will reduce serum cholesterol levels. The reported mechanism by which sterols reduce serum cholesterol is through the disruption of intestinal absorption of dietary cholesterol by displacing it from bile acid micelli. More recently, β-sitosterol's saturated equivalent, β-sitostanol, has been shown to be more effective in the reduction of intestinal cholesterol absorption. Furthermore, β-sitostanol itself is virtually unabsorbed, so it does not contribute at all to *in vivo* serum cholesterol concentration following consumption.

There have been reports that describe how the esterification of sterols (in this specification the term sterol will be used to refer to either sterols or stanols) with a fatty acid or an edible oil produces a sterol ester with improved solubility characteristics. It has also been suggested that these low melting lipophilic esters are more soluble in the micelle phase during the digestive process. These esters have been incorporated effectively into, for example, margarine, for use as a palatable dietary supplement.

Studies have shown that plant sterols are most effective at reducing serum cholesterol levels when taken three times per day every day for at least two weeks. There is an unmet consumer need for a convenient, portable, palatable dosage form for these ingredients which can be consumed anywhere without the need for food or water.

The properties of phytosterols and especially those of sterol esters make them particularly unsuited toward formulation into traditional pharmaceutical dosage forms. Typical dosing regimens require ingestion of relatively large amounts, which would require the consumer to swallow several tablets or capsules to get a single dose. The poor wetability of these compounds makes them difficult to incorporate into aqueous based forms such as liquids. Additionally, their low melting point and waxy properties make them difficult to process with conventional pharmaceutical manufacturing equipment.

### Summary of the Invention

We have found that sterols and sterol esters may be successfully incorporated into confectionery dosage forms with excellent palatability. This approach results in a convenient, palatable, portable dosage form for reduction of serum cholesterol. An unexpected advantage of formulating the sterols and sterol esters in confections is that they impart unique and desirable organoleptic properties when added to a confectionery matrix. The unique properties include decreased adhesion to the teeth, faster melt-away, softer texture, and shorter bite. Perhaps more advantageous and unexpected, was the finding that the stanol and sterol and preferably their esters may be incorporated into the confectionery formulae in place of all or part of the typically required fats without adversely effecting the organoleptic properties of the confection. During processing, the cholesterol-lowering agent acts as a lubricant, facilitates cutting, and imparts many of the properties traditionally achieved by including fats in the formulation.

The present invention provides for a confectionery matrix that contains a cholesterol lowering agent, such as stanol/sterol and its corresponding esters. More particularly, the present invention provides confectionery matrices that can be chewed or sucked, and easily swallowed, preferably without food or water.

### Detailed Description of the Invention

The present invention provides a product with excellent palatability and mouthfeel characteristics. The active stanol, sterol or stanol/sterol ester is dispersed in a confectionery matrix. The confectionery matrix is comprised of a carbohydrate, water and the cholesterol lowering agent. Optionally other ingredients are incorporated into the confectionery matrix such as, but not limited to emulsifiers, fat, colors, flavors, sweeteners and the like.

Suitable matrices include candy forms such as chocolate, taffy, nougat, fudge, caramel, hard candy, gummies, and the like, as well as filling materials for chocolates, doughnuts, or confectionery bars, such as cremes, jellies, peanut butter, etc. As used herein gummies are understood to be a the well known candy formed from a solid material made from stiffening agent such as starch, gelatin and pectin; a high amount of water such as from about 10 to about 20 weight percent, and a sweetener or flavoring agent.

The principle constituent of the matrix is typically a carbohydrate. The carbohydrate can be sugar, such as sucrose, glucose, fructose, lactose and the like, or sugar alcohols, such as sorbitol, mannitol, xylitol, maltitol, lactitol, isomalt and the like, or starch derivatives such as maltodextrins, dextrins, and the like. The preferred carbohydrates are sugars. The sugar component commonly comprises a mixture of a monosaccharide and a disaccharide. For example, a common source of a disaccharide is sucrose, and a common source of a monosaccharide, is glucose, which is also known in the art as dextrose. Com syrup is often employed as a source of glucose. It is well known in the art that the relative proportion and properties of these sweeteners plays a major role in determining the physical characteristics of the product.

In some confectionery forms, excluding the cholesterol-lowering agent and some water, sugar may employed at a level up to about 100 weight %. Typically the sugar component is from about 50 to 100% and preferably from about 65-98 weight % on a dry solids basis.

The level of disaccharide, such as sucrose, in the present invention is up to about 75 weight percent and typically comprises from about 25-65% on a dry solid basis. More preferably the level is from about 35-65% on a dry solid basis. The level of monosaccharide , such as glucose derived from corn syrup, is up to about 50 weight percent preferably up to about 40 weight percent and in a highly preferred embodiment the syrup is about 35% on a dry solid basis. Sugar alcohols or other carbohydrates may be substituted for both the disaccharide and monosaccharide components.

The carbohydrate content of corn syrup is indicated by the dextrose equivalent (D.E.). The dextrose equivalent as used herein is understood as the percent of the reducing sugar calculated as dextrose on a dry substance basis. It is the carbohydrate composition that controls the functional properties and therefore the choice of syrups to be used. Generally, the lower the D.E. of the corn syrup, the chewier the product will be and the less sweet to taste. As the D.E. increases, the sweetness increases and the chewiness lessens. Com syrup D.E. ranges from about 20 through about 68; the corn syrup industry has defined 17 functional properties of corn syrup as they relate to D.E.

The cholesterol lowering agents employed in the present invention include sitosterols, sitostanol, the esters of the sterol/stanol materials and mixtures of these materials. For example, β-sitosterols are typically derived from wood or agricultural sources, such as soy based mixtures. In addition to β-sitosterol, as used throughout this application, β-sitosterol is also understood to include the esters of β-sitosterols, as well as stanol and stanol ester derivatives which are the reduced derivatives of the sterols. These derivatives are well known in the art and include US Patents 5,244,887; and US 5,698,527. Methods for producing these esters are also known in the art, see for example US Patent 5,502,045 and German 2,035,069.

Another advantage of the present invention is that unlike the disclosure of WO 99/15546, published April 1, 1999, the stanol and sterol esters which may be incorporated into the confectionery matrix may be substantially non-polar. As is well known in the art, an ester is commonly structurally depicted as RC(O)OR'. The aforementioned patent application disclosure teaches that ester groups that contain polar molecules such as oxygen, attached to the sterol and stanol rings such that the esters are soluble in lipid and ethanol and dispersible in water. The hydroxyl group of the sterol/stanol ring is esterified at the 3β-position of the sterol/stanol ring with a polar substituent, for example a aliphatic hydroxy ketone, ketoacids, dicarboxlic acids or amino acids. Surprisingly, the present invention provides for non-polar sustituents, that is groups that contain only hydrogen and carbon atoms, to the sterol/stanol structures and which can be incorporated into the confectionery matrix. As used in the ester formula depicted above R' is understood to include aliphatic straight or branched carbon chains having a length of about C₃-C₂₄, preferably from C₆-C₂₂ and most preferably C₁₂-C₂₁ groups, and R₂ is understood to include aliphatic straight or branched carbon chains ranging C₃-C₁₅, preferably C₆-C₁₂, and most preferably, C₉ groups. More preferably, R₂ is selected from the group (C₁-C₁₂) alkyl, (C₂-C₈) alkenyl, (C₂-C₈) alkynyl, (C₃-C₈) cycloalkyl, halo (C₂-C₈) alkenyl, halo (C₂-C₈) alkynyl) where halo is understood to include chloro, fluoro, bromo, iodo and the like. Alkyl includes both straight and branched chain groups of carbon atoms. Typical alkyl groups include methyl, ethyl, n-propyl, isopropyl, n-butyl, sec-butyl, isobutyl, t-butyl, n-pentyl, neopentyl, isopentyl, hexyl, heptyl and the like. The alkyl groups may be halogenated with one, two three or more halogen atoms. The terms alkenyl and alkynyl included branded and straight chain hydrocarbons having at least one unsaturated bond.

The present invention is also applicable to another class of cholesterol-lowering compounds, orynzanol and related compounds as well as mixtures of these compounds. These materials are also known in the art, see for example, U.S. Patent No. 5,514,398, the contents hereby incorporated by reference, and PCT application W098/01519 published January 15, 1998.

The present invention incorporates an effective amount of cholesterol-lowering compound in the sugar-based matrix. Typical levels are from 0.5 to about 20 grams per day, preferably from about 2 to about 15 grams per day and most preferably from about 4 to about 10 grams per day of the cholesterol-lowering agent. Commonly the cholesterol-lowering agent is provided in an amount of about 2 grams per serving which is consumed three or more times per day.

A further advantage of the present invention is that the cholesterol lowering agents do not need to be ground to a microcrystalline size, that is less than about 35 microns as disclosed in WO 98/13023. The plant sterols as used in this invention can be greater than about 100 microns, preferably greater than about 200 and most preferably greater than about 300 microns in size and still be effectively incorporated into the confectionery matrix. Those with skill in the art appreciate that smaller particle size materials are more easily dispersed or distributed throughout a matrix. However, it has been surprisingly discovered that larger size particles of the cholesterol-lowering agent can be incorporated into the present invention without adversely effecting product quality.

In some embodiments of the invention an emulsifier is optionally added to the sugar matrix in order to enhance the physical properties of the sugar matrix for incorporation of an oily type material. The addition of the emulsifier to the sugars typically reduces the stickiness of the sugars, improves the mold release characteristics, decreases sticking to the teeth, improves the mouthfeel and improves the ability to chew and swallow the product. As used herein, an emulsifier is meant to mean any material that will emulsify an oil when added with water. Suitable emulsifiers include both synthetic and natural origins, including but not limited to, lecithin, glyceryl esters, sugar esters, polyglycerol esters, polysorbates, mono and diglycerides of fatty acids, propylene glycol esters, sucrose fatty acid esters and polyoxyethylene derivatives of sorbitan fatty acid esters. These emulsifiers are well known in the art and are commercially available.

Other useful emulsifiers include polysorbates made from the reaction product of monoglycerides or sorbitan esters with ethylene oxides. Examples of useful polysorbates include sorbitan trioleate, sorbitan monopalmitate, sorbitan monolaurate, propylene glycol monolaurate, glycerol monostearate, diglycerol monostearate, glycerol lactyl-palmitate. Lactic acid derivatives include sodium stearoyl lactylate and calcium stearoyl lactylate.

As is appreciated by those with skill in the art, the HLB value for a emulsifier is an expression of its Hydrophile-Lipophile balance, i.e., the balance of the size and strength of the hydrophilic (polar) and lipophilic (non-polar) groups of the emulsifier. When using the optional emulsifiers, varying levels of emulsifiers have been discovered that will possess beneficial emulsifying properties in this system. The emulsifier level is up to about 10 weight percent, preferably from about 0.1 to about 5 and most preferably from about 0.15 to about 5 weight percent. For example, when using a β-sitostanol ester at 12% dry weight basis, the proper concentration of emulsifier is about 0.34%. Those skilled in the art will recognize that other emulsifiers can be used in combination with a specified sugar/syrup ratio, e.g., a mixture of 2 or more emulsifiers, without departing from the present invention.

The product of the invention may optionally incorporate a saturated fat or partially hydrogenated vegetable oil. A preferred hydrogenated vegetable oil is partially hydrogenated cottonseed oil having a melting point of from about 95°F to about 150°F. Preferred saturated fats are palm oil and butter. The ratio of the sterol to the partially hydrogenated vegetable oil may vary over a broad range. In a preferred embodiment, the sterol is used in place of all or part the vegetable oil.

The confectionery matrices of the present invention may contain varying levels of fat content. For example, in some candies, such as but not limited to boiled candies, the candy is substantially fat-free. As used herein, substantially fat free is understood to be more than about 90 weight percent, preferably greater than about 95, most preferably greater than 99 and in a highly preferred embodiment the candy contains no fat. Other confectioneries such as but not limited to hard boiled toffee have a higher fat content up to about 24 weight percent, preferably about 10 to about 20 weight percent. Some candies have even higher fat content, for example, chocolate candies have higher levels of fat, that is up to about 35 weight percent.

Another surprising advantage of the present invention is that it has been discovered that the cholesterol lowering agent, particularly the sterol, stanol and the related esters can be employed in the present invention, and that a reduced fat confectionery can be produced. The present invention provides for the replacement of some of the fat by the cholesterol lowering agent, for example, sterol/stanol and its esters. It has been surprisingly discovered that the weight of fat in the confectionery matrix is replaced by the cholesterol lowering agent. Preferably, the cholesterol lowering agent replaces an equal weight of fat in the confectionery, however, the replacement ratio of cholesterol lowering agent to fat ranges from about 1:10 to about 10:1.

The present invention may also include materials such as pigments, colorants, preservatives, flavorings, and mixtures of these in varying amounts. These ingredients are generally incorporated in the comestible product up to about 7 weight percent, and more preferably up to about 3.5 weight percent of the final product. Preservatives or antioxidants such as, but not limited to, sorbic acid, benzoic acid, butylatedhydroxytoluene (BHT), butylatedhydroxyanisole (BHA), vitamin E, ascorbic acid, selenium, lycopene, dihydroxyacetone (DHA) or β-carotene may also be incorporated. Auxiliary sweeteners may also be employed, either natural or synthetic. Flavorings include both natural and artificial flavors and include mints such as: peppermint, spearmint, and menthol; cinnamon, chocolate, caramel, vanilla, fruit flavors and mixtures of flavors.

Water is used to dissolve the sugar and makes up the remainder of the product weight. The water content of the finished product will influence its physical and organoleptic properties, and will vary widely depending on the particular confectionery form. Water is present in the final product at levels typically less than about 40 weight percent, preferably less than about 20 weight percent, and most preferably at a level from about 3 to 15 weight %.

One advantage of the present invention is that the sugar matrix has a texture and mouthfeel similar to candy, soft, easily chewed or sucked and swallowed. In a preferred embodiment of the invention, no additional fat is required to be added to the sugar matrix in order to provide the desired mouth feel and texture.

A further advantage of the present invention is the desirable organoleptic properties imparted by the sterols, such as reduced adhesion to the teeth, faster melt-away, softer smoother texture, and shorter bite.

In a preferred embodiment of the present invention for making a nougat, the sugar component comprises a disaccharide, which is typically sucrose, and a monosaccharide, which is typically glucose (dextrose) from com syrup. The level of sucrose is typically from about 20 to about 60, preferably from about 25 to about 45, and more preferably from about 35 to about 40. The level of com syrup is typically from about 25 to about 50, preferably from about 30 to about 40, and more preferably from about 32 to about 36. The dextrose equivalent of the corn syrup employed is typically from about 20 to about 68+, preferably from about 38 to about 58, and more preferably from about 42 to 43 D.E. A D.E. of about 42 to about 43 enhances mouthfeel, texture and tender chewing characteristics of the invention.

The preferred embodiment for making a nougat typically employs an emulsifier. Preferred emulsifiers include lecithin and glyceryl monostearate. The level of emulsifier in the nougat is typically from about 0.1 to about 10, preferably from about 0.2 to about 6, and more preferably from about 0.25 to about 5.

The preferred embodiment for making a nougat also incorporates an effective amount of an aerating agent in the sugar matrix. Methods for making chewable comestible products via an aerating agent, kn own as frappé, is known in the art, see for example US Pat. 4,545,989 hereby incorporated by reference. Preferred aerating agents include whipped egg whites, soy albumin and milk protein. The amount of aerating agent will determine the amount of air incorporated into the product and change the physical characteristics of the product. The level of aerating agent employed in this embodiment is related to physical characteristics producing the desired mouthfeel and texture. The level of aerating agent used in the nougat embodiment is typically from about 5 to about 25%, preferably from about 10 to about 20% and most preferably from about 12.5 to about 17.5% of the product on a dry weight basis.

Confectionery nougats typically contain a partially hydrogenated vegetable oil or saturated fat. A preferred vegetable oil is partially hydrogenated cottonseed oil having a melting point of from about 95°F to about 150°F. Preferred saturated fats are palm oil and butter. The level of fat in a confectionery nougat is typically from about 1% to about 8% by weight of dry solids. In the present invention, the sterol or sterol ester is used in place of all or part the fat. In a highly preferred embodiment for making a nougat, the cholesterol-lowering agent is used to substantially replace the fat. The ratio of the sterol to the partially hydrogenated vegetable oil may vary over a broad range. In the preferred embodiment for making a nougat, the level of fat is typically less than about 5 weight percent, generally less than about 3 weight percent, preferably less than about 2 weight percent, and most preferably less than about 1 weight percent.

The cholesterol-lowering agent acts as a lubricant, facilitates cutting and adds mouthfeel that is typically imparted by fats. It also helps to reduce stickiness. Typically the cholesterol-lowering agent is employed in the sugar matrix at a level dependent on the dosage strength desired. In a preferred nougat form, the level of sterol or stanol is typically from about 1 to about 30, preferably from about 4 to about 20, and more preferably from about 6 to about 12% by weight of dry solids. In a preferred nougat form, the level of sterol or stanol ester is typically from about 1 to about 40, preferably from about 6 to about 25, and more preferably from about 8 to about 14% by weight of dry solids.

Water is used to dissolve the sugar and makes up the remainder of the product weight. Water is present in the final nougat product at levels less than about 20 weight percent, preferably from about 3 to 15 weight % and most preferably at a level from about 7 to 10 weight %.

The preferred nougat form may contain additional minor ingredients commonly employed in the art, such as flavoring agents, salts, colors, and the like.

The preferred process for preparing a nougat comprises the following steps. The sugars, emulsifier, and cholesterol lowering agent is added to water to form a sugar suspension. The sugar suspension is then heated. After the water fraction of the sugar suspension is reduced to the desired solid content, the sugar suspension is then cooled to approximately 210°F, and the aerating agent is added to the sugar suspension. At a cooler temperature that still allows flowability of the sugar suspension, it is appropriate to add flavoring, artificial sweeteners, coloring, food acids, preservatives and the like. The contents of the mixture are then mixed to achieve a uniform composition.

In order to form the desired final shape of the matrix several options are possible. First the mixture can be allowed to cool sufficiently such that the mixture could be extruded and cut into desired length pieces. In a preferred method, the mixture is transferred to a cooling slab and cut into pieces.

The chewable solid matrix of the preferred nougat form has a texture and hardness such that the texture is chewed and swallowed in less than 20 seconds, preferably in less than 15 seconds and most preferably in less than about 10 seconds. While the sugar matrix of the preferred nougat is rapidly chewed and swallowed, the texture of the matrix is such that it does not appreciably dissolve in the mouth in the times stated above.

The present invention additionally encompasses a hard boiled candy composition comprising a confectionery base and a cholesterol lowering agent, such as stanol/sterol or its corresponding esters. Hard boiled candy compositions generally have a candy base composed of a mixture of sugar and other carbohydrate bulking agents, with the following characteristics: non-crystalline, clear and glassy appearance, extremely low amount of residual moisture from about 0.5% to about 3.0% moisture, and a variable amount of invert sugar, monosaccharides, the result of partial inversion of sucrose resulting from the cooking process.

In a preferred embodiment for making a hard boiled candy composition, the base may contain disaccharide, preferably sucrose, in amounts of up to **99**% and monosaccharide, preferably glucose from syrup in amounts up to about 70% syrup. The level of sucrose is typically from about 40 to about **99**, preferably from about 50 to about 80, and more preferably from about 60 to about 70. The level of syrup is typically from about 0 to about 70, preferably from about 20 to about 50, and more preferably from about 30 to about 40.

Further ingredients may be added to satisfy consumer taste and texture as well as improve esthetic appearance such as flavoring agents, sweeteners, acidulants, colorants, milk, milk protein, cream, butter, honey, malt extract, etc. Some of these ingredients may incidentally add nutritive value. Additionally, raw materials may be used to modify rheological properties of the sugar mass to obtain a particular texture, to reduce viscosity in process, reduce raw material cost, reduce sweetness, reduce stickiness, effect Maillard reaction or provide sugar-free product. These may include dextrose, lactose, sorbitol, mannitol, xylitol, maltitol, isomalt, erythritol, hydrogenated starch hydrolysates, fats and emulsifiers and the like.

Water is used to dissolve the sugar and makes up the remainder of the product weight. Water is present in the final hard boiled candy product at levels less than about 10 weight percent, preferably from about 1 to 5 weight % and most preferably at a level from about 2 to 4 weight %.

An advantage of the present invention is the desirable organoleptic properties imparted by the sterols, such as reduced adhesion to the teeth, faster melt-away, softer smoother texture, and shorter bite of the hard boiled candy product.

The following examples are provided as specific embodiments of the present invention. Other modifications of this invention will be readily apparent to those skilled in these arts without departing from the scope of the present invention. The following commercially available materials were used in the examples that follow. The glyceryl monostearate (GMS) was Atlas 1500 obtained from ICI. The glucose com syrup was 43 D.E. com syrup obtained from Cargill. The sucrose was obtained from Domino. The frappé used was Marshmallow Creme obtained from Favorite Brands International, β-sitostanol (stanol) and it's rape seed oil derived ester (stanol-ester) were obtained from Rasio. Ingredients as whipping cream, butter, salt and sweetened condensed milk are commonly available.

### Example 1 - Chewy Nougat

The following formulations were prepared:

| | | |
|---|---|---|
| A) | Sucrose | 38.2% Dry solids |
| | Corn Syrup | 34.1 % Dry solids |
| | GMS | 0.34% Dry solids |
| | Stanol Ester | 12.0% Dry solids |
| | Marshmallow Creme | 15.3% Dry solids |
| | | |
| B) | Sucrose | 37.5% Dry solids |
| | Corn Syrup | 33.4% Dry solids |
| | GMS | 0.34% Dry solids |
| | Stanol- Ester | 12.0% Dry solids |
| | Marshmallow Creme | 15.3% Dry solids |
| | Cocoa Powder | 1.38% Dry solids |
| | | |
| C) | Sucrose | 40.8% Dry solids |
| | Corn Syrup | 36.2% Dry solids |
| | GMS | 0.34% Dry solids |
| | Stanol | 7.2% Dry solids |
| | Marshmallow Creme | 15.4% Dry solids |

The chewy nougat was prepared by the following method:

Purified water (approximately 25% by weight of the amount of sucrose) and corn syrup were added to a sauce pan. Sucrose was then added followed by glyceryl monostearate. Stanol or stanol-ester was then added, the mixture stirred and placed on a hot plate. With constant stirring, the sugar suspension was heated above boiling. The suspension was heated with stirring until cooked down to the desired percent solids. The mixture was then allowed to cool down to a suitable temperature at which the marshmallow creme was folded in. The contents of the mixture were then mixed to achieve a uniform concentration. While the mixture was still warm enough to flow, it was deposited onto a foil lined pan and allowed to flow to an even thickness and depth. After tempering overnight, the mixture was cut into pieces of the appropriate weight to assure active content uniformity.

The finished nougat products from all three formulas (A, B, and C) were sweet and pleasant tasting with no undesirable ("off") flavors or aftertastes. All three products chewed easily with no adhesion to the teeth or packing in the molars. All three products could be chewed and swallowed in less than 10 seconds, with good clear away, leaving a clean palate.

### Example 2 - Hard Boiled Non-Fat Candy

The following two formulations were prepared:

| Ingredient | Placebo Formula (% dry solids) | Stanol Formula (% dry solids) |
|---|---|---|
| Water | 16 | 15 |
| Sucrose | 65 | 60.8 |
| Corn Syrup | 35 | 32.8 |
| Stanol | - | 6.4 |

The hard boiled non-fat candy was prepared by the following method:
Purified water and sucrose were added to a sauce pan and placed on a hot plate. Sucrose was dissolved and brought to a boil. Corn syrup was then added, mixed, brought to a boil, covered to steam 2 minutes, then uncovered. Stanol was then added, dispersed, and the entire mixture cooked to a temperature - 225-250°F. The suspension was heated with stirring until cooked down to the desired percent solids. The mixture was then allowed to cool down to a suitable temperature at which the flavoring was incorporated. The contents of the mixture were then mixed to achieve a uniform concentration. While the mixture was still warm enough to flow, it was deposited as quarter-sized drops onto foil and allowed to cool.

Both products were sweet and pleasant tasting with no "off' flavors. The stanol containing hard candy had noticably less adhesion to the teeth, and was easier to chew.

### Example 3 - Hard Boiled Candy with Fat

| | Placebo Formula (% dry solids) | Stanol Formula (% dry solids) |
|---|---|---|
| Sucrose | 40 | 36.5 |
| Corn Syrup | 38.9 | 36.0 |
| Butter | 11.5 | 11.5 |
| Sweetened Condensed Milk | 7.5 | 7.5 |
| GMS | 0.16 | 0.16 |
| Salt | 0.63 | 0.63 |
| Whipping Cream | 1.2 | 1.2 |
| Stanol | -- | 6.3 |

The hard boiled candy with fat was prepared by the following method:
Purified water (approximately 25% by weight of the amount of sucrose) and sucrose were added to a sauce pan and placed on a hot plate. Sucrose was dissolved and brought to a boil. Corn syrup was then added, mixed, brought to a boil, and cooked to a temperature about285°F. Separately in another pan, sweetened condensed milk, butter, whipping cream, GMS and salt were mixed and heated on a separate hot plate to about 230°F. The fat mixture was then added to the sugar base and mixed well. Stanol was then added, dispersed, and the entire mixture cooked to a temperature ∼ 250°F. The suspension was heated with stirring until cooked down to the desired percent solids. The mixture was then allowed to cool down to a suitable temperature at which the flavoring was incorporated. The contents of the mixture were then mixed to achieve a uniform concentration. While the mixture was still warm enough to flow, it was deposited as quarter-sized drops onto foil and allowed to cool.

Both products were sweet and pleasant tasting with no "off" flavors. The stanol containing version had noticibly less adhesion to the teeth, as well as a smoother texture, shorter bite, and faster dissolution in the mouth, and was easier to chew.

## Claims

1. An orally administerable composition comprising:
a confectionery matrix;
an effective amount of a cholesterol-lowering agent.

2. The composition of claim 1 wherein the cholesterol-lowering agent is a stanol, sterol, sterol-ester, stanol-ester or orynzanol.

3. The composition of claim 1 or claim 2 wherein the cholesterol-lowering agent(s) is (are) added to a hard boiled candy.

4. The composition of any one of claims 1 to 3 wherein the confectionery matrix comprises sucrose and glucose from corn syrup which has a dextrose equivalent of from 30 to 45.

5. The composition of any one of claims 1 to 4 wherein the confectionery matrix is a filling material for candy bars, chocolate cremes, doughnuts or pastries.

6. The composition of any one of claims 1 to 5 wherein the cholesterol-lowering agent is present in an amount from 1% to 40% of the dry weight of the composition.

7. The composition of any one of claims 1 to 6 wherein the cholesterol-lowering agent is provided in an amount from 0.1 to 20 grams per piece.

8. The composition of any one of claims 1 to 7 wherein the level of cholesterol-lowering agent is from 1 to 50% by weight of dry solids in the composition.

9. The composition of any one of claims 1 to 8 which is substantially fat free or which is substantially free of vegetable oils or which is substantially sugar free.

10. The composition of any one of claims 1 to 8 which further contains a fat; wherein the weight ratio of cholesterol-lowering agent to fat is from 1:10 to 10:1.
